Europäisches Patentamt

European Patent Office

Office européen des brevets

(19)

(11) **EP 0 682 243 B1**

(12) **EUROPÄISCHE PATENTSCHRIFT**

(45) Veröffentlichungstag und Bekanntmachung des Hinweises auf die Patenterteilung:
**27.08.2003 Patentblatt 2003/35**

(51) Int Cl.7: **G01N 21/25**, G01N 21/31, G01N 21/47

(21) Anmeldenummer: **95104506.1**

(22) Anmeldetag: **27.03.1995**

(54) **Vorrichtung und Verfahren zur Messung des Bräunungsgrades eines Gargutes**

Equipment and procedure for the measurement of the degree of browning of baked products

Dispositif et procédé pour la mesure du degré du cuisson de ferment alimentaire

(84) Benannte Vertragsstaaten:
**AT BE CH DE DK ES FR GB GR IT LI LU NL PT SE**

(30) Priorität: **14.05.1994 DE 4416762**

(43) Veröffentlichungstag der Anmeldung:
**15.11.1995 Patentblatt 1995/46**

(73) Patentinhaber: **BSH Bosch und Siemens Hausgeräte GmbH
81669 München (DE)**

(72) Erfinder:
• **Dausch, Manfred, Dipl.-Ing. (FH)
D-76831 Göcklingen (DE)**
• **Fey, Dirk, Dipl.-Ing. (FH)
D-76199 Karlsruhe (DE)**
• **Krieg, Gunther, Prof. Dr.-Ing.
D-76227 Karlsruhe (DE)**
• **Koukolitschek, Karl, Dipl.-Ing.
D-76189 Karlsruhe (DE)**
• **Maier, Wilfried
D-75056 Sulzfeld (DE)**
• **Braun, Martin
D-73760 Ostfildern (DE)**

(74) Vertreter: **Richter, Harald, Dr. et al
BSH Bosch und Siemens Hausgeräte GmbH
Zentralabteilung
Patente und Lizenzen
Hochstrasse 17
81669 München (DE)**

(56) Entgegenhaltungen:
EP-A- 0 244 721     DE-A- 3 540 751
DE-A- 3 615 259     DE-B- 2 616 546
GB-A- 2 098 725     US-A- 4 886 366
US-A- 5 229 841

**Beschreibung**

Die Erfindung betrifft eine Vorrichtung und ein Verfahren zur Messung des Bräunungsgrades eines Gargutes.

Backwaren und andere Lebensmittel werden in Bäckereibetrieben, in der Gastronomie u.s.w. meist in Öfen mittels fest programmierter Zeit-Temperatur-Programme hergestellt. Dabei wird eine empirisch ermittelte Temperatur und Backzeit für die betreffenden Backwaren zugrunde gelegt, d.h. dabei werden spezielle Backzeiten und Backtemperaturen für die jeweiligen Backwaren verwendet.

Als nachteilig hat sich hierbei gezeigt, daß die betreffenden Backprogramme lediglich empirisch für die einzelnen Backwaren ermittelt werden und somit nur annähernd der gewünschte Garzustand erzielt werden kann. Es versteht sich, daß natürlich Größen- und Massenabweichungen, Veränderungen der Teigzusammensetzung und auch auf den Ofen zurückgehende Parameter den Garzustand und den Bräunungsgrad der Backwaren beeinflussen können.

Pyrometrische Verfahren und andere Meßverfahren, die grundsätzlich für eine derartige Messung zur Verfügung stehen, lassen sich kaum für eine derartige Messung verwenden, da das Meßergebnis zu sehr von zufälligen Einflußfaktoren, wie der Anordnung des Gargutes im Garraum und ähnlichen Dingen beeinflußt wird.

Der Begriff "Bräunungsgrad" wird nachfolgend allgemein derart verstanden, daß hierunter eine Veränderung der Oberflächenfärbung erfaßt wird, die sich von anfangs ganz hell bis später zu dunkleren Werten hin verändert. Ein Bräunungsgrad von 0 würde dabei theoretisch einer weißen Farbe entsprechen, während ein Bräunungsgrad von 1 theoretisch einer schwarzen Fläche entsprechen würde.

Aus der GB-A-2 098 725 sind ein Verfahren und eine Vorrichtung zur Online-Messung von Lebensmittelparametern bekannt geworden, wonach verschiedene Parameter, wie etwa die Farbe, der Feuchtigkeitsgehalt, der Fettgehalt und der Zuckergehalt bestimmt werden können. Hierzu wird ein Vergleich von modulierter Infrarot-Strahlung bei mindestens zwei Wellenlängen gemacht, die auf das Material gelenkt wird. Die Strahlung der einzelnen Strahlungsquellen wird über Strahlteiler aufgeteilt, um mittels eines Referenzdetektors die Stärke der von den Strahlungsquellen emittierten Strahlung zu detektieren, während der andere Teil der Strahlung über Fokussierlinsen auf die Lebensmittel gerichtet wird. Die von den Lebensmitteln reflektierte Strahlung wird über eine Fokussiereinrichtung auf einen Meßdetektor gelenkt und ausgewertet. Aus dem Vergleich der Meßsignale werden unter Berücksichtigung von geeigneten Standards die gewünschten Parameter des Lebensmittels bestimmt.

Im vorliegenden Fall handelt es sich allerdings lediglich um eine Meßeinrichtung zur Eigenschaftsbestimmung an kalten Lebensmitteln, die nicht geeignet ist, um an einem Gargut innerhalb einer Ofenatmosphäre einen Bräunungsgrad zu bestimmen.

Der Erfindung liegt somit die Aufgabe zugrunde, eine Vorrichtung und ein Verfahren zur Messung des Bräunungsgrades eines Gargutes zu schaffen, womit zuverlässige Meßergebnisse auch unter Bedingungen, wie sie in einer Ofenatmosphäre herrschen, erzielt werden können.

Diese Aufgabe wird erfindungsgemäß durch eine Vorrichtung mit den Merkmalen des Anspruchs 1 gelöst.

Erfindungsgemäß werden Lichtwellenleiter zur Übertragung von Strahlung von der Strahlungsquelle in den Garraum und zur Übertragung von Strahlung aus dem Garraum zu den Sensoren verwendet.

Dadurch können die notwendigen Sensoren zur Erfassung der abgestrahlten Strahlung in den unterschiedlichen Wellenlängenbereichen und zur Erfassung der rückgestrahlten Strahlung in den zugeordneten Wellenlängenbereichen vom Garraum entkoppelt werden.

Zwar steht für Transmissionsmessungen das sogenannte Vier-Strahl-Dual-Wellenlängen-Verfahren gemäß der DE-B-3 615 259 und gemäß der EP-B-0 244 721 zur Verfügung, wonach eine Referenzwellenlänge und eine Meßwellenlänge verwendet werden, um nach dem Beerschen Gesetz die Konzentration eines Stoffes im Transmissionverfahren zu bestimmen, jedoch ist die Anwendung eines derartigen Verfahrens der Messung des Bräunungsgrades eines Gargutes bei einer Wärmebehandlung in einem Garraum nicht ohne weiteres möglich, da hierbei keine einfache Transmissionsmessung durchgeführt werden kann, sondern die vom Bräunungsgrad des Gargutes beeinflußte Reflektion und Rückstreuung gemessen werden muß.

Erfindungsgemäß wird es erstmalig ermöglicht, den Bräunungsgrad eines Gargutes, insbesondere eines Backgutes meßtechnisch zu erfassen. Durch die Anwendung des Vier-Strahl-Dual-Wellenlängen-Verfahrens, das die Intensitäten von mindestens zwei Strahlungsquellen unterschiedlicher Wellenlängen-Emissionsbereiche auswertet, deren Reflektion und Rückstreuung unterschiedlich vom Bräunungsgrad des Gargutes beeinflußt wird, wird der Einfluß der Geometrie, insbesondere von Art, Menge und Anordnung des Gargutes im Garraum und Entfernung des Gargutes vom Meßwertaufnehmer, von Störgrößen, der schwankenden Gaszusammensetzung und Luftfeuchtigkeit, der Einfluß von Driftproblemen, von Schwankungen der Strahlungsquellen, des Transmissionsvermögens der optischen Anordnung, des Einflusses von Verschmutzungen des Garraums und der optischen Anordnung weitgehend eliminiert. Somit wird erfindungsgemäß auf einfache Weise eine präzise Messung des Bräunungsgrades des Gargutes ermöglicht. Im Unterschied zu den bekannten Meßverfahren gemäß der DE-B-3 615 259 und der EP-B-0 244 721 wird nicht die Transmission zur Messung herangezogen, sondern statt dessen wird die reflektierte, rückgestreute Strahlung zweier unterschiedlicher Wellenlängen, die durch den Bräunungsgrad des Gargutes unterschiedlich beeinflußt wird, zur Messung ausgewertet.

Ausgehend von dem Lambert Beer'schen Gesetz: $I = I_0 . e^{-kd}$ mit k als Absorptionskoeffizient, d als Schichtdicke des absorbierenden Stoffes, wird in Anlehnung an die sogenannte dekadische Extinktion folgender Auswertealgorithmus zur Bestimmung des Bräunungsgrades B herangezogen.

$$B = \log_{10} \left( \frac{\dfrac{I_{Meß,RD}}{I_{Meß,MD}}}{\dfrac{I_{Ref,RD}}{I_{Ref,MD}}} \right) \tag{1}$$

mit

B =         Bräunungsgrad

$I_{Meß,RD}$ =     vom Referenzsensor erfaßte Intensität der Meßstrahlung

$I_{Meß,MD}$ =     vom Meßsensor erfaßte Intensität der Meßstrahlung

$I_{Ref,RD}$ =     vom Referenzsensor erfaßte Intensität der Referenzstrahlung

$I_{Ref,MD}$ =     vom Meßsensor erfaßte Intensität der Referenzstrahlung

Durch die Verwendung dieses Algorithmus werden insbesondere die komplizierten Geometrieeinflüsse ausgeschlossen, die bisher eine Messung des Bräunungsgrades bei beliebiger Verteilung des Gargutes im Garraum unmöglich machten. Ferner werden Meßwertebeeinträchtigungen ausgeschlossen, die durch Verschmutzung, Trübung, Reflektion, Intensitätsänderungen der Strahlenquellen usw. verursacht werden.

Dabei kann auf eine aufwendige Logarithmierstufe verzichtet werden, indem der im allgemeinen nicht lineare Zusammenhang direkt als Wertetabelle oder als analytische Funktion, z.B. als Polynom n-ten Grades, in einem Speicher abgelegt wird, der sich im Zugriffsbereich der elektronischen Signalauswertung befindet. Dabei kann ferner zur Erhöhung der Meßgenauigkeit vor Beginn jedes Meßzyklus eine Aufnahme der Gerätedunkelwerte (ohne Meßstrahlung und ohne Referenzstrahlung bei leerem Garraum) erfolgen. Diese Gerätedunkelwerte werden dann von den betreffenden Intensitäten vor der Quotientenbildung abgezogen.

Eine weitere Vereinfachung der Auswertung kann dadurch erreicht werden, daß die Intensität der Meßstrahlung und der Referenzstrahlung mittels der vom Referenzsensor erfaßten Strahlungsintensitäten auf einem konstanten Wert gehalten wird.

Eine besonders einfache Auswertung ergibt sich in vorteilhafter Weiterbildung der Erfindung mit einer Einrichtung zur Beendigung der Meßzeit der Meßstrahlung in Abhängigkeit vom Empfangszeitpunkt einer vorgebbaren empfangenen Strahlungsenergie der Referenzstrahlung, und mit einer Einrichtung zur Integration des Meßsignals der Intensität der Meßstrahlung am Meßdetektor über die Meßzeit zur Bestimmung des Meßergebnisses.

Auf diese Weise kann auf eine Logarithmierstufe und auf eine Quotientenbildung verzichtet werden; es sind statt dessen lediglich einfache Zähler erforderlich. vorzugsweise werden dabei die Referenzstrahlung und die Meßstrahlung unterschiedlich moduliert und zueinander zeitlich versetzt getaktet erzeugt und die von dem Meßdetektor und dem Referenzdetektor aufgenommenen Meßsignale über eine Schalteinrichtung einer gemeinsamen Verstärker- und Filtereinheit zur Verarbeitung zugeführt, die frequenzselektiv arbeitet und so mit einem einheitlichen Verstärkungsfaktor eine Verstärkung der nachher wieder trennbaren Signale ermöglicht, die der Meßsensor von der Meßstrahlung und der Referenzstrahlung und der Referenzsensor von der Meßstrahlung und der Referenzstrahlung aufnehmen.

In bevorzugter Weiterbildung der Erfindung wird die Referenzstrahlung und die Meßstrahlung über einen Strahlteiler in zwei Teilstrahlenbündel aufgespalten, wobei das erste Teilstrahlenbündel dem Referenzsensor zur Erfassung der Intensitäten der Referenzstrahlung und der Meßstrahlung zugeführt ist, und wobei das zweite Teilstrahlenbündel der Meßstrahlung und der Referenzstrahlung über die Optik in den Garraum eingestrahlt wird.

Durch die Aufteilung der emittierten Strahlung mittels des Strahlteilers, der z.B. als teilweise reflektierendes Prisma ausgebildet sein kann, in das erste Strahlenbündel zur Erfassung der Intensitäten der Referenzstrahlung und der Meßstrahlung, und in das zweite Teilstrahlenbündel zur Einstrahlung der Referenzstrahlung und der Meßstrahlung über die Optik in den Garraum, wird erfindungsgemäß auf einfache Weise eine Erfassung der in den Garraum eingestrahlten Intensitäten der Meßstrahlung und der Referenzstrahlung ermöglicht.

Wie zuvor bereits erwähnt, wird das Meßsignal des Referenzsensors dabei vorzugsweise einer Einrichtung zur Regelung der Strahlungs intensitäten der Meßstrahlung und der Referenzstrahlung auf einen konstanten Wert zugeführt.

In besonders bevorzugter Ausführung der Erfindung wird das zweite Teilstrahlenbündel der Referenzstrahlung und der Meßstrahlung über mindestens einen Lichtwellenleiter der Optik zugeführt, von der die Referenzstrahlung in einer Ebene aufgefächert etwa in der Höhe des Gargutes in den Garraum eingestrahlt wird, wobei die vom Gargut reflektierte und rückgestreute Strahlung über mindestens einen im wesentlichen außerhalb des Strahlungsbereiches der in den Garraum einfallenden Meßstrahlung und Referenzstrahlung angeordneten Lichtwellenleiter dem Meßsensor zugeführt wird.

Auf diese Weise wird über den mindestens einen Lichtwellenleiter mit der von der Optik bewirkten linearen Auffächerung der Meßstrahlung und der Referenzstrahlung eine gleichmäßige Bestrahlung des Gargutes erreicht. Da der mindestens eine Lichtwellenleiter, der zum Empfang der vom Gargut infolge der einfallenden Referenzstrahlung und Meßstrahlung emittierten Strahlung vorgesehen ist, im wesentlichen außerhalb des Strahlungsbereiches der in den Garraum einfallenden Meßstrahlung und Referenzstrahlung angeordnet ist, wird ein erheblich verbesserter Signal/ Rauschabstand erreicht.

In weiter vorteilhafter Ausgestaltung der Erfindung ist jeweils ein Lichtwellenleiter vorgesehen, um,die Meßstrahlung und die Referenzstrahlung vom Strahlteiler zur einer Optik zu übertragen, die beide etwa mittig über die Optik aufgefächert in den Garraum einstrahlen, wobei seitlich in den Randbereichen des Garraums je ein Lichtwellenleiter vorgesehen ist, um die vom Gargut reflektierte und rückgestreute, über die Optik gebündelte Strahlung dem Meßsensor zuzuführen.

Mit dieser Ausführung ergibt sich ein besonders einfacher Aufbau, da lediglich zwei Lichtquellen unterschiedlicher Wellenlängenbereiche erforderlich sind, von denen die Referenzstrahlung und die Meßstrahlung über die beiden Lichtwellenleiter zur Optik übertragen werden, die eine ausreichend breite Auffächerung bewirkt, um ein gleichmäßiges Bestrahlen des im Garraum angeordneten Gargutes zu ermöglichen. Um eine ausreichende Bestrahlungsintensität sicherzustellen, werden vorzugsweise Lichtwellenleiter mit einem Durchmesser von etwa 2 mm verwendet. Die beiden Lichtwellenleiter zum Empfang der vom Gargut reflektierten, rückgestreuten und von der Optik gebündelten Strahlung weisen vorzugsweise einen kleineren Durchmesser in der Größenordnung von etwa 1 mm auf und sind seitlich in den Randbereichen des Garraums angeordnet, um eine Entkopplung von der in den Garraum eingestrahlten Strahlung sicherzustellen.

Es versteht sich, daß an Stelle von zwei einzelnen Lichtquellen etwa in der Form von LEDs auch eine einzige konventionelle Lichtquelle verwendet werden kann, der geeignete Filter vorgeschaltet sind.

Daneben kann gemäß einer Weiterbildung der Erfindung auch ein lineares Lichtquellenarray verwendet werden, dessen Lichtenergie in ein ebenfalls linear angeordnetes Lichtfaserbündel eingekoppelt wird. Auch hierbei wird ein Teil der vom Lichtquellenarray abgestrahlten Referenz- und Meßsignale über ein weiteres Faserteilbündel zum Referenzsensor umgelenkt, um die Intensität der in den Garraum eingestrahlten Meßstrahlung und Referenzstrahlung zu kontrollieren bzw. auf einen konstanten Wert zu regeln.

Eine derartige Anordnung ist zwar etwas aufwendiger als die zuvor erwähnte, hat jedoch den Vorteil, daß sich eine noch höhere Meßgenauigkeit erzielen läßt, da durch unterschiedliche Längen der Lichtwellenleiter bedingte Einflüsse vermieden werden. Auch kann eine noch gleichmäßigere Bestrahlung des Garraums erreicht werden. Vorzugsweise sind die verschiedenfarbigen LEDs des Arrays abwechselnd nebeneinander angeordnet.

In bevorzugter Weiterbildung der Erfindung ist die Optik als linearer Hohlspiegel oder als Zylinderlinse ausgebildet.

Damit läßt sich, ausgehend von einer Lichtquelle mit kleinem Austrittswinkel, wie sie ein Lichtwellenleiter darstellt, eine lineare Auffächerung in einer Ebene erreichen.

Zur Ermittlung des Bräunungsgrades bei Backgut liegt die Meßstrahlung vorzugsweise im gelben sichtbaren Bereich und die Referenzstrahlung vorzugsweise im Infrarotbereich.

Dabei kann die Meßstrahlung und die Referenzstrahlung von Lichtdioden (LEDs) erzeugt werden, die im Wellenlängenbereich von etwa 600 Nanometer und etwa 1000 Nanometer emittieren.

Es versteht sich, daß in Abhängigkeit von zu messendem Gargut auch andere Wellenlängenbereiche für die Referenzstrahlung und die Meßstrahlung bevorzugt sein können.

In bevorzugter weiterbildung der Erfindung werden der Referenzsensor und der Meßsensor durch einen thermischen Kurzschluß auf der gleichen Temperatur gehalten, um Temperatureinflüsse auf das Meßergebnis auszuschalten.

Die erfindungsgemäße Vorrichtung ist mit einer Ofensteuerung, über die die Heizung des Garraums gesteuert wird, derart kombiniert, daß eine Abschaltung der Heizeinrichtung bei Erreichen eines vorbestimmten Bräunungsgrades erreicht wird.

Somit kann das betreffende Gargut jeweils bis zu einem bestimmten Bräunungsgrad gegart werden, woraufhin dann eine automatische Abschaltung des Ofens erreicht wird.

Die Aufgabe der Erfindung wird ferner durch ein Verfahren gëmäß Anspruch 16 gelöst.

Wie bereits zuvor erwähnt, wird dabei vorzugsweise das Meßsignal der Intensität der Referenzstrahlung am Referenzsensor über eine solche Meßzeit integriert, bis ein fest vorgebbarer Wert des Integrals erreicht ist und das Meßsignal der Intensität der Meßstrahlung am Meßdetektor über die gleiche Meßzeit integriert und das zum Ende dieser Meßzeit erreichte Integral des Meßwerts der Intensität der Meßstrahlung am Meßdetektor zur Bestimmung des Meßergebnis des herangezogen.

Es versteht sich, daß die vorstehend genannten und die nachfolgend noch zu erläuternden Merkmale der Erfindung nicht nur in der jeweils angegebenen Kombination, sondern auch in anderen Kombinationen oder in Alleinstellung verwendbar sind, ohne den Rahmen der Erfindung zu verlassen, wie dieser durch die Ansprüche definiert ist.

Weitere Merkmale und Vorteile der Erfindung ergeben sich aus der nachfolgenden Beschreibung eines bevorzugten Ausführungsbeispiels unter Bezugnahme auf die Zeichnung. Es zeigen:

Fig. 1 eine erfindungsgemäße Vorrichtung in vereinfachter Darstellung;

Fig. 2 eine alternative Ausführung der erfindungsgemäßen Vorrichtung in stark vereinfachter, schematischer Darstellung;

Fig. 3 eine vergrößerte Darstellung der Lichtleiteranordnung gemäß Fig. 2, wobei die Optik jedoch als Zylinderlinse ausgeführt ist;

Fig. 4 ein Diagramm des mit der erfindungsgemäßen Vorrichtung aufgenommenen Bräunungsgrades von Bretzeln in Abhängigkeit von der Backzeit;

Fig. 5 ein Diagramm des mit der erfindungsgemäßen Vorrichtung aufgenommenen Bräunungsgrades von Spitzbrötchen in Abhängigkeit von der Backzeit und

Fig. 6 ein Diagramm des mit der erfindungsgemäßen Vorrichtung aufgenommenen Bräunungsgrades von Laugenbrötchen in Abhängigkeit von der Backzeit

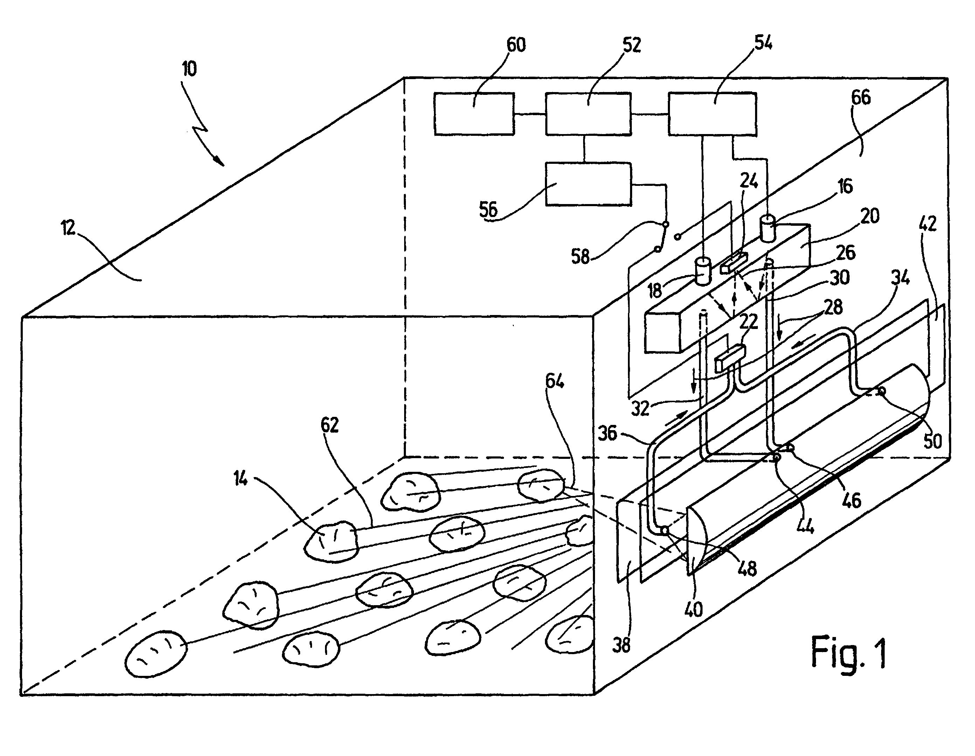

In Fig. 1 ist eine erfindungsgemäße Vorrichtung insgesamt mit der Ziffer 10 bezeichnet.

Im dargestellten Beispiel ist die erfindungsgemäße Vorrichtung 10 als Ofen mit einem Garraum 12 ausgeführt, in dem sich Gargut befindet, das schematisch mit der Ziffer 14 angedeutet ist. Es versteht sich, daß das Gargut, wie etwa Bretzeln, Spitzbrötchen, Laugenbrötchen und ähnliche Backwaren in der Praxis auf mehreren Einschüben angeordnet sein kann. Darüber hinaus könnte es sich bei dem Gargut auch etwa um Fleischstücke, wie Bratenstücke, um Würstchen oder dergleichen handeln, deren Bräunung erfindungsgemäß erfaßt wird.

Bei der erfindungsgemäßen Vorrichtung 10 wurde auf die Darstellung der bei herkömmlichen Öfen üblichen Komponenten, wie die Auskleidung des Garraums, Umluftventilator, Heizung, Isolierung, Tür, Bedienungselemente, Kondensatablauf usw. verzichtet, da diese nicht Gegenstand der vorliegenden Erfindung sind.

Die erfindungsgemäße Vorrichtung 10 weist zwei Strahlungsquellen 16, 18 in Form von Hochleistungs-LEDs auf, von denen die eine 16 eine Meßstrahlung im Bereich von etwa 600 Nanometer abstrahlt und die andere 18 eine Referenzstrahlung von etwa 1000 Nanometer abstrahlt.

Die beiden Strahlungsquellen 16, 18 werden von einer Treibereinheit gesteuert, die schematisch mit der Ziffer 54 angedeutet ist. Die Strahlung der beiden Strahlungsquellen 16, 18 wird unmittelbar in einen Strahlteiler 20 eingekoppelt, von dem ein Teil der eingekoppelten Referenzstrahlung und Meßstrahlung als ein erstes Teilstrahlenbündel 26 unmittelbar von einem Referenzsensor 24 erfaßt wird.

Der andere, größere Teil der von den Strahlungsquellen 16 und 18 emittierten Strahlung wird über je einen Lichtwellenleiter 30 und 32 mit einem relativ großen Durchmesser von etwa 2 mm unmittelbar zu einer Optik 40 übertragen, die im dargestellten Beispiel als linearer Hohlspiegel ausgebildet ist.

Dabei sind die beiden Lichtwellenleiter 30, 32 mit ihren Austrittsöffnungen 44, 46 derart an einer durchsichtigen Befestigungsplatte 42 befestigt, daß sie unmittelbar nebeneinander etwa in der Brennebene des linearen Hohlspiegels in diesen einstrahlen. Durch den Hohlspiegel 40 wird die von den Lichtwellenleitern 30, 32 übertragene Energie breit in einer Ebene aufgefächert auf das Gargut 14 durch ein entsprechendes Fenster 38 in der Seitenwand 66 eingestrahlt, wie schematisch durch die Einfallsstrahlen 62 angedeutet ist.

Die auf das Gargut einfallende Strahlungsenergie führt zur Reflektion und Rückstreuung von Strahlung, die von der oberflächenbeschaffenheit, insbesondere vom Bräunungsgrad des Gargutes 14 beeinflußt wird. Die vom Gargut 14 zurückgestrahlte Strahlungsenergie gelangt wiederum durch das Fenster 38, das beispielsweise aus einem Borosilikat-Glas - wie etwa Duran (eingetragenes Warenzeichen der Firma Schott) bestehen kann, wieder zum Hohlspiegel 40 gelangt, wie durch die gestrichelten emittier - ten Strahlen 64 beispielhaft bei einem Teil des Gargutes 14 dargestellt ist.

Die reflektierten, rückgestreuten Strahlen 64 werden durch den Hohlspiegel 40 gebündelt und gelangen zu zwei an den Enden des Hohlspiegels 40 gleichfalls an der Befestigungsplatte 42 befestigten Lichtwellenleitern 34 bzw. 36, deren Eintrittsöffnungen 48, 50 sich wiederum etwa in der Brennebene des Hohlspiegels 40 befinden. Die so gebündelte Energie der vom Gargut 14 reflektierten, rückgestreuten Strahlen wird über die beiden Lichtwellenleiter 34, 36 zu einem gemeinsamen Meßsensor 22 übertragen, der die Intensität der Strahlung erfaßt. Dadurch daß die beiden Lichtwellenleiter 34, 36 mit ihren Eintrittsöffnungen 48, 50 seitlich in den Randbereichen des Garraums 12 und an den Enden des Hohlspiegels 40 angeordnet sind, wird eine gute Entkopplung der in den Garraum 12 eingestrahlten Strahlung von der vom Gargut 14 reflektierten, rückgestreuten Strahlung 64 erreicht, so daß ein gutes Signal/Rauschspannungsverhältnis erreicht wird.

Der Meßsensor 22 und der Referenzsensor 24 werden vorzugsweise durch einen thermischen Kurzschluß auf der gleichen Temperatur gehalten, um einen Temperatureinfluß auf die Messung auszuschließen. Hierzu können beide Sensoren beispielsweise wassergekühlt sein.

Es versteht sich, daß die Optik in Abwandlung von der in Fig. 1 dargestellten Ausführung statt als Hohlspiegel auch als Zylinderlinse ausgebildet sein kann, wobei dann natürlich die Eintritts- bzw. Austrittsenden der Lichtwellenleiter nicht zwischen dem Garraum 12 und der Optik 40 angeordnet sind, sondern vom Garraum 12 aus gesehen hinter der Optik.

Die von den beiden Strahlungsquellen 16, 18 emittierte Meßund Referenzstrahlung wird von der Treibereinheit 54 mit unterschiedlichen Wellenlängen hochfrequent moduliert, wobei die Referenzstrahlung und die Meßstrahlung zueinander seitlich versetzt getaktet sind. Die vom Meßsensor 22 und vom Referenzsensor aufgenommenen Meßsignale werden, wie schematisch durch die Ziffer 58 angedeutet, über eine Schalteinheit einer gemeinsamen Verstärkungs- und Filtereinheit 56 zugeführt, die eine Verstärkung und frequenzselektive Filterung der vom Meßsensor 22 und vom Referenzsensor 24 aufgenommenen Signale der Referenzstrahlung und der Meßstrahlung mit einem einheitlichen Verstärkungsfaktor ermöglicht. Durch die Modulation mit unterschiedlichen Frequenzen läßt sich anschließend wieder eine Trennung der Signale durchführen. Die Verstärkungs- und Filtereinheit 56 ist wie auch die Treibereinheit 54 mit einer Steuereinheit 52 gekoppelt, die mit Unterstützung eines Mikroprozessors eine Steuerung der gesamten Meßeinrichtung und eine Auswertung der Meßergebnisse zur Ableitung des gewünschten Bräunungsgrades übernimmt.

Auf eine Beschreibung der Einzelkomponenten der Elektronik kann an dieser Stelle verzichtet werden, da diese im einzelnen in der EP-B-0 244 721 beschrieben ist, auf die in diesem Zusammenhang Bezug genommen wird.

Die Steuereinheit 52 ist mit einer durch die Ziffer 60 angedeuteten Ofensteuerung gekoppelt, über die beispielsweise eine Abschaltung der Heizung erfolgen kann, sobald ein vorbestimmter Bräunungsgrad für ein bestimmtes Gargut erreicht wird. Das vom Referenzsensor 24 aufgenommene Meßsignal des ersten Strahlteilenbündels 26 aus der Referenzstrahlung und der Meßstrahlung wird dazu verwendet, um die Intensitäten der Strahlungsquellen 16, 18 auf einen vorbestimmten Wert zu regeln, so daß eine konstante Strahlungsintensität sowohl der Referenzstrahlung als auch der Meßstrahlung während der Gesamtdauer des Garvorgangs sichergestellt ist.

Der Bräunungsgrad B wird erfindungsgemäß nach folgendem Auswertungsalgorithmus berechnet:

$$B = \log_{10} \left( \frac{\dfrac{I_{Meß,RD}}{I_{Meß,MD}}}{\dfrac{I_{Ref,RD}}{I_{Ref,MD}}} \right) \qquad\qquad (1)$$

mit

B = Bräunungsgrad
$I_{Meß,RD}$ = vom Referenzsensor erfaßte Intensität der Meßstrahlung
$I_{Meß,MD}$ = vom Meßsensor erfaßte Intensität der Meßstrahlung
$I_{Ref,RD}$ = vom Referenzsensor erfaßte Intensität der Referenzstrahlung
$I_{Ref,MD}$ = vom Meßsensor erfaßte Intensität der Referenzstrahlung

Um eine größere Meßgenauigkeit zu erreichen und die Einflüsse von Gerätewerten auszuschalten, kann zusätzlich vor der Inbetriebnahme des Ofens jeweils ein sogenannter Gerätedunkelwert oder Offset-Wert bei leerem Ofen aufgenommen werden, ohne daß die beiden Strahlungsquellen 16, 18 eingeschaltet sind. Diese Offset-Werte für die Meßstrahlung und die Referenzstrahlung werden dann von den jeweiligen vom Referenzsensor und vom Meßsensor erfaßten Intensitäten (Gleichung 1) subtrahiert.

Eine Normierung der Meßwerte auf einen Bräunungsgrad von 1 bei einem schwarzen Körper kann beispielsweise mittels eines geschwärzten Probekörpers durchgeführt werden.

Da die Auswertung der Meßsignale durch Integration erfolgt, derart, daß das Meßsignal der Referenzstrahlung am Meßdetektor über eine solche Meßzeit integriert wird, bis ein fest vorgebbarer Wert des Integrals erreicht ist, und das Meßsignal der Meßstrahlung am Meßdetektor über die gleiche Meßzeit integriert wird, kann das zum Ende dieser Meßzeit erreichte Integral des Meßwerts der Meßstrahlung am Meßdetektor als Meßergebnis herangezogen werden, wobei auf eine Logarithmierung und Quotientenbildung verzichtet werden kann, da das Meßergebnis als doppelspaltige Tabelle in einem Speicher abgelegt werden kann, die nach einer entsprechenden Eichung unmittelbar den Bräunungsgrad wiedergibt.

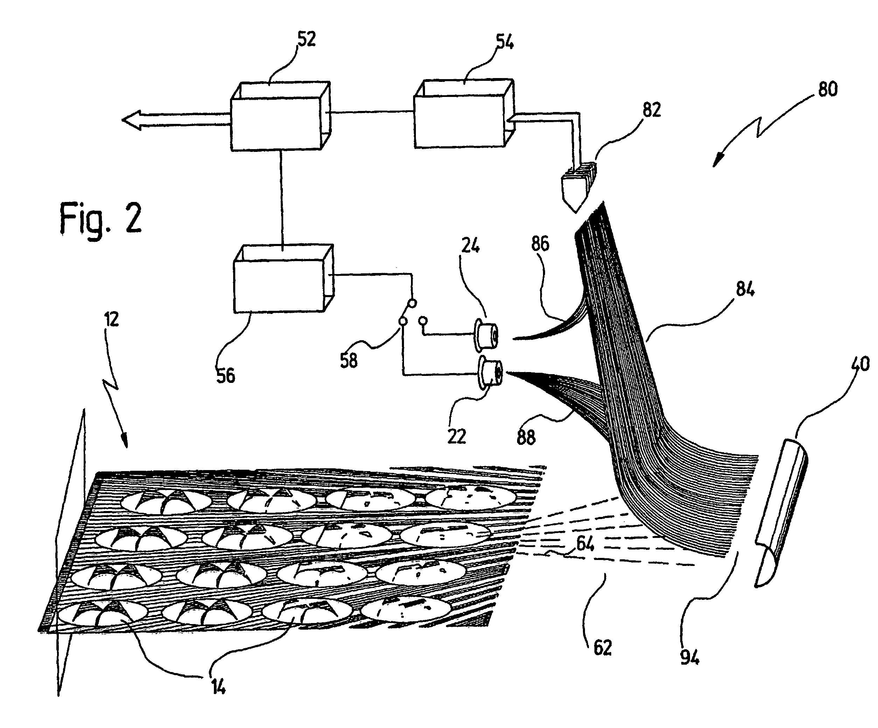

In Fig. 2 ist eine gegenüber der Fig. 1 abgewandelte Ausführung der Erfindung äußerst schematisch dargestellt und insgesamt mit der Ziffer 80 bezeichnet.

Der zuvor erläuterten Ausführung entsprechende Teile sind dabei mit denselben Bezugsziffern bezeichnet.

Der wesentliche Unterschied zu der zuvor anhand von Fig. 1 erläuterten Ausführung besteht darin, daß nicht zwei einzelne Strahlungsquellen verwendet werden, sondern daß stattdessen ein lineares Beleuchtungsarray 82 verwendet wird. Die von diesem Beleuchtungsarray 82 erzeugte Meßstrahlung und Referenzstrahlung wird durch ein geeignet aufgebautes lineares Lichtwellenleiterbündel 84 zur Optik 40 übertragen. Von der Meß- und Referenzstrahlung wird wiederum ein kleiner Teil über ein erstes Teilfaserbündel 86 abgezweigt und dem Referenzsensor 24 zugeführt. Die vom Gargut 14 reflektierte und zurückgestreute Strahlung, die wiederum schematisch durch die Ziffer 64 angedeutet ist, wird nach der Bündelung durch die Optik 40 von einem zweiten Teilfaserbündel 88 zum Meßsensor 22 übertragen.

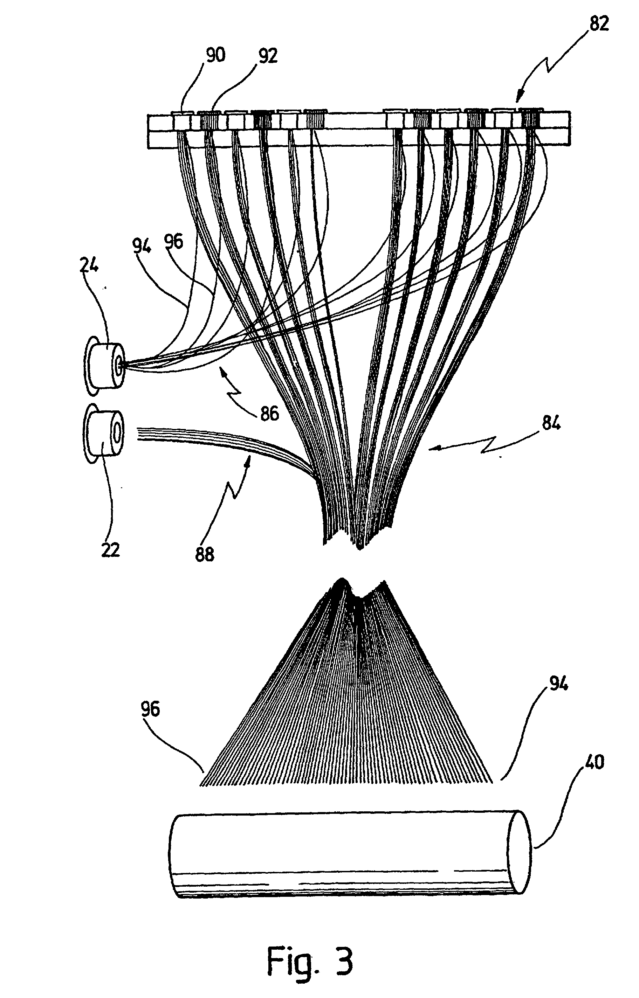

Der genauere Aufbau des Beleuchtungsarrays 82 und der Lichtwellenleiterbündel ist aus Fig. 3 zu ersehen, die eine vergrößerte schematische Darstellung der optischen Komponenten gemäß Fig. 2 zeigt.

In Abwandlung von der Ausführung gemäß Fig. 2 ist die Optik 40 nicht als linearer Hohlspiegel, sondern als Zylinderlinse ausgeführt.

Das Beleuchtungsarray 82 weist eine Reihe von nebeneinander angeordneten LEDs 90, 92, auf, die die Referenzstrahlung bzw. die Meßstrahlung emittieren. Die die Referenzstrahlung bzw. die Meßstrahlung emittierenden LEDs 90 bzw. 92 sind alternierend hintereinander angeordnet. Die von jeder LED 90, 92 ausgesandte Strahlung wird über ein in geeigneter Weise angekoppeltes Lichtwellenleiterbündel 84 zur Optik 40 übertragen.

Ein Teil der von den LEDs 90, 92 emittierten Referenzstrahlung bzw. Meßstrahlung wird durch das Teilfaserbündel 86 zum Referenzsensor 24 übertragen. Hierzu reicht es aus, wenn jeder LED 90, 92 ein einzelner Lichtwellenleiter 94 bzw. 96 zugeordnet ist, um einen Bruchteil der emittierten Energie zum Referenzsensor 24 zu übertragen.

Die Austrittsenden 94 des Lichtwellenleiterbündels 84 und die Eintrittsenden des Teilfaserbündels 88 sind vor der Optik 40 etwa in deren Brennebene zeilenförmig nebeneinander angeordnet, wobei die Austrittsenden 94 und die Eintrittsenden 96 in gleichmäßiger Verteilung durchmischt angeordnet sind, so daß die Strahlung jeder Leuchtdiode 90, 92 gleichmäßig räumlich verteilt auf das Gargut 14 fällt und die vom Gargut reflektierte, gestreute Strahlung repräsentativ für das gesamte Gargut vom Meßsensor 22 erfaßt werden kann.

Durch diese Anordnung wird eine gegenüber der zuvor anhand von Fig. 1 beschriebenen Anordnung verbesserte Meßgenauigkeit erreicht, da eine gleichmäßigere räumliche Verteilung der Bestrahlung erreicht wird und die Einzellängen der Lichtwellenleiter des emittierenden Lichtwellenleiterbündels 84 und des empfangenden Teilfaserbündels 88 jeweils gleich sind, so daß dadurch bedingte Meßfehler vermieden werden.

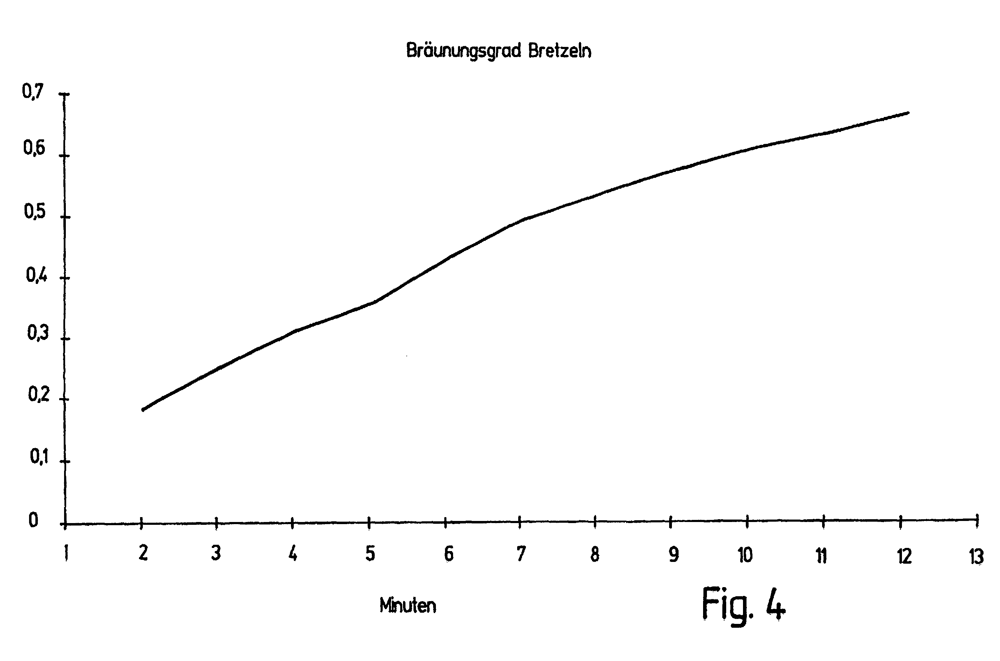

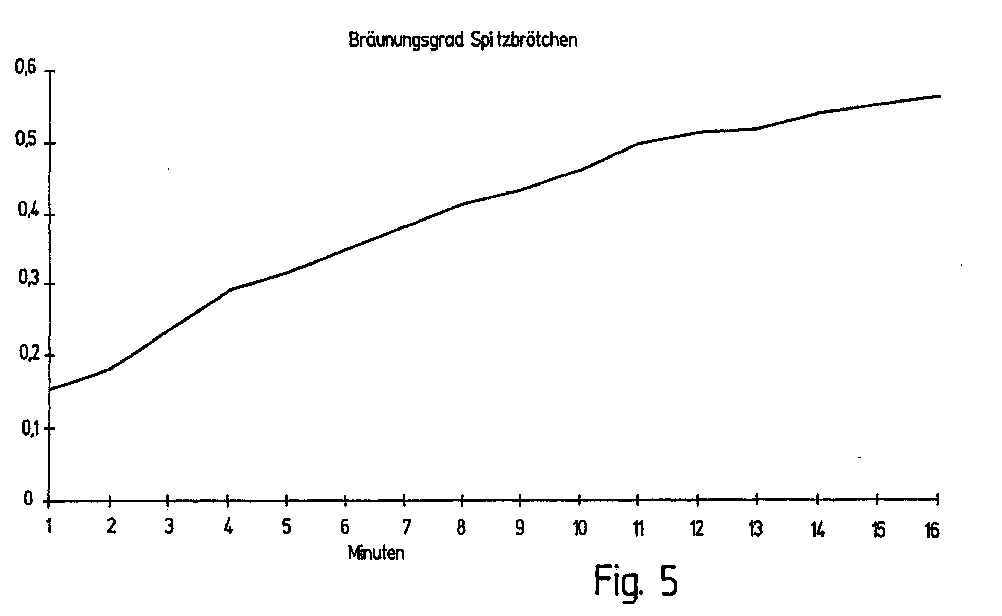

Die Figuren 4, 5 und 6 zeigen beispielhaft drei Darstellungen für den ermittelten Bräunungsgrad in Abhängigkeit von der Backzeit bei Bretzeln, bei Spitzbrötchen und bei Laugenbrötchen.

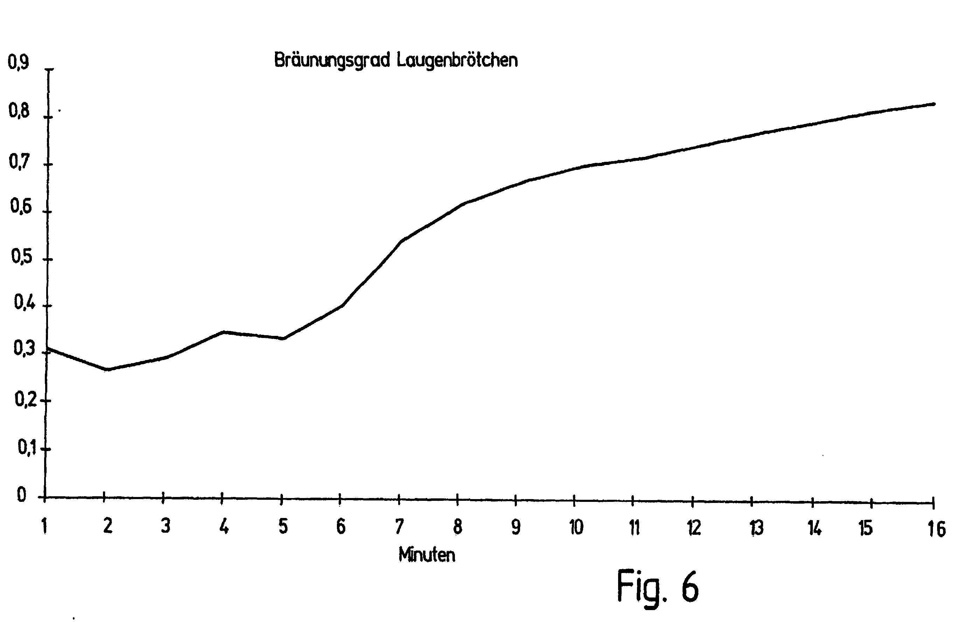

Die Welligkeit der Kurven (vgl. insbesondere Fig. 6) ist dabei auf Formänderungen und insbesondere auf das "Aufbrechen" der betreffenden Backgüter zurückzuführen.

Bei den dargestellten Versuchsergebnissen war das Backblech mit 6 bis 8 Stück des jeweiligen Backguts belegt. Der Abstand der, von der Optik aus gesehen, vordersten Backstücke lag bei etwa 15 cm.

## Patentansprüche

**1.** Vorrichtung zur Messung des Bräunungsgrades eines Gargutes (14), insbesondere zur Messung des Bräunungsgrades eines Backgutes, mit einem Garraum (12), mit mindestens einer Strahlungsquelle (16, 18; 82), die eine Meßstrahlung, die eine Strahlung eines ersten Wellenlängenbereiches ist und eine Referenzstrahlung, die eine Strahlung eines zweiten, davon verschiedenen Wellenlängenbereiches ist, erzeugt, die beide in den Garraum (12) abgestrahlt werden, mit einem Meßsensor (22) zur Erfassung einer ersten Intensität ($I_{Meß,MD}$) von aus dem Garraum (12) aufgenommener Strahlung im ersten Wellenlängenbereich und einer zweiten Intensität ($I_{Ref,MD}$) von aus dem Garraum (12) aufgenommener Strahlung im zweiten Wellenlängenbereich, und mit einem Referenzsensor (24), der mit der Strahlungsquelle (16, 18) gekoppelt ist zur. Erfassung einer dritten Intensität ($I_{Meß,RD}$) von in den Garraum (12) abgestrahlter Strahlung im ersten Wellenlängenbereich und zur Erfassung einer vierten Intensität ($I_{Ref,RD}$) von in den Garraum (12) abgestrahlter Strahlung im zweiten Wellenlängenbereich, und mit einer Einrichtung (52) zur Ermittlung des Bräunungsgrades aus den von den Sensoren (22, 24) gemessenen ersten, zweiten, dritten und vierten Intensitäten ($I_{Meß,MD}$, $I_{Ref,MD}$, $I_{Meß,RD}$, $I_{Ref,RD}$), wobei Lichtwellenleiter (30, 32, 34, 36; 84, 86, 88) zur Übertragung von Strahlung von der Strahlungsquelle (16, 18; 82) in den Garraum (12) und zur Übertragung von Strahlung aus dem Garraum ( 12 ) zu dem Meßsensor (22) vorgesehen sind, und mit einer ofensteuerung (60), der der Bräunungsgrad einer Ofensteuerung (60) zur Steuerung einer Wärmebehandlung des Garraumes zugeführt ist.

2. Vorrichtung nach Anspruch 1, bei der die von der Strahlungsquelle (16, 18) emittierte Meßstrahlung und Referenzstrahlung einem Strahlteiler (20) zur Aufspaltung in zwei Teilstrahlenbündel (26, 28) zugeführt ist, wobei das erste Teilstrahlenbündel (26) dem Referenzsensor (22) zur Erfassung der dritten und vierten Intensitäten ($I_{Meß,RD}$, $I_{Ref,RD}$) zugeführt ist, und wobei das zweite Teilstrahlenbündel (28) der Meßstrahlung und der Referenzstrahlung in den Garraum (12) eingestrahlt wird.

3. Vorrichtung nach Anspruch 2, bei der das vom Referenzsensor (24) erhaltene Meßsignal der dritten und vierten Intensität ($I_{Meß, RD}$, $I_{Ref, MD}$) einer Einrichtung (54) zur Regelung der Strahlungsintensitäten der emittierten Meßstrahlung und der emittierten Referenzstrahlung auf einen konstanten Wert zugeführt ist.

4. Vorrichtung nach einem der vorhergehenden Ansprüche, bei der das zweite Teilstrahlenbündel (28) der Referenzstrahlung und der Meßstrahlung über mindestens einen Lichtwellenleiter (30, 32) einer Optik (40) zugeführt ist, von der die Referenzstrahlung in einer Ebene aufgefächert etwa in der Höhe des Gargutes (14) in den Garraum (12) eingestrahlt wird, und von der die aus dem Garraum (12) reflektierte, rückgestreute Strahlung über mindestens einen im wesentlichen außerhalb des Strahlungsbereiches der in den Garraum (12) einfallenden Meßstrahlung und Referenzstrahlung angeordneten Lichtwellenleiter (36, 38) dem Meßsensor (22) zugeführt ist.

5. Vorrichtung nach Anspruch 4, bei der jeweils ein Lichtwellenleiter (30, 32) vorgesehen ist, um den Strahlteiler (20) mit der Optik (40) zu verbinden, wobei beide Lichtwellenleiter (30, 32) angeordnet sind, etwa mittig über die Optik (40) aufgefächert in den. Garraum (12) einzustrahlen, und wobei seitlich in den Randbereichen des Garraums (12) je ein Lichtwellenleiter (34, 36) vorgesehen ist, der die aus dem Garraum (12) reflektierte, rückgestreute, über die Optik (40) gebündelte Strahlung dem Meßsensor (22) zuführt.

6. Vorrichtung nach einem der vorhergehenden Ansprüche, bei der als Strahlungsquelle ein lineares Lichtquellenarray (82) vorgesehen ist, dessen Strahlung über ein Lichtwellenleiterbündel (84) in den Garraum (12) eingekoppelt wird.

7. Vorrichtung nach Anspruch 6, bei der ein Teil der von dem Lichtquellenarray (82) emittierten Strahlung über ein Teilfaserbündel (86) einem Referenzsensor (24) zugeführt ist, bei der die restliche emittierte Strahlung über die Optik (40) breit aufgefächert in den Garraum (12) eingestrahlt wird, und bei der die von der Optik (40) gebündelte, aus dem Garraum (12) reflektierte und rückgestreute Strahlung über ein zweites Teilfaserbündel (88) dem Meßsensor (22) zugeführt ist.

8. Vorrichtung nach Anspruch 6 oder 7, bei der die die Referenzstrahlung und die Meßstrahlung übertragenden Lichtwellenleiter und die die reflektierte und rückgestreute Strahlung übertragenden Lichtwellenleiter weitgehend gleichmäßig verteilt, zeilenförmig angeordnet sind.

9. Vorrichtung nach einem der Ansprüche 4 bis 8, bei der die Optik (40) als linearer Hohlspiegel oder als Zylinderlinse ausgebildet ist.

10. Vorrichtung nach einem der vorhergehenden Ansprüche, bei der die Meßstrahlung im gelben sichtbaren Bereich und die Referenzstrahlung im Infrarotbereich liegt.

11. Vorrichtung nach Anspruch 10, bei der die Meßstrahlung und die Referenzstrahlung von LEDs (16, 18) erzeugt wird, die im Wellenlängenbereich von etwa 600 nm und etwa 1000 nm emittieren.

12. Vorrichtung nach Anspruch 1-11 bei der die Ofensteuerung (60) eine Abschaltung einer Heizeinrichtung bei Erreichen eines vorbestimmbaren Bräunungsgrades bewirkt.

**13.** Vorrichtung nach einem der vorhergehenden Ansprüche, mit einer Einrichtung (60) zur Beendigung der Meßzeit der Meßstrahlung in Abhängigkeit vom Empfangszeitpunkt einer vorgebbaren empfangenen Strahlungsenergie der Referenzstrahlung, und mit einer Einrichtung (52) zur Integration des Meßsignals der Intensität der Meßstrahlung am Meßdetektor über die Meßzeit zur Bestimmung des Meßergebnisses.

**14.** Vorrichtung nach einem der vorhergehenden Ansprüche, bei der die Referenzstrahlung und die Meßstrahlung mit unterschiedlichen Frequenzen moduliert und zueinander zeitlich versetzt getaktet erzeugt werden, und bei der die von dem Meßsensor und dem Referenzsensor aufgenommenen Meßsignale über eine Schalteinrichtung (58) einer gemeinsamen Verstärker- und Filtereinheit (56) zur Verarbeitung zugeführt sind.

**15.** Verfahren zur Messung des Bräunungsgrades eines Gargutes (14), insbesondere zur Messung des Bräunungsgrades eines Backgutes, bei einer Wärmebehandlung in einem Garraum (12), bei dem von mindestens einer Strahlungsquelle (16, 18; 82) eine Meßstrahlung, die eine Strahlung eines ersten Wellenlängenbereiches ist, und eine eine Referenzstrahlung, die Strahlung eines zweiten, davon verschiedenen Wellenlängenbereiches ist, erzeugt wird, die beide in den Garraum (12) abgestrahlt werden, mit einem Meßsensor (22) eine erste Intensität ($I_{Meß,MD}$) von aus dem Garraum (12) aufgenommener Strahlung im ersten Wellenlängenbereich und eine zweite Intensität ($I_{Ref,MD}$) von aus dem Garraum (12) aufgenommener Strahlung im zweiten Wellenlängenbereich erfaßt wird, bei dem mittels eines mit der Strahlungsquelle (16, 18; 82) gekoppelten Referenzsensors (24) eine dritte Intensität ($I_{Meß,RD}$) von in den Garraum (12) abgestrahlter Strahlung im ersten Wellenlängenbereich und eine vierte Intensität ($I_{Ref,RD}$) von in den Garraum (12) abgestrahlter Strahlung im zweiten Wellenlängenbereich erfaßt wird, wobei die Strahlung zwischen dem Garraum (12) und den Sensoren (22, 24) über Lichtwellenleiter (30, 32, 34, 36; 84, 86, 88) übertragen wird und wobei der Bräunungsgrad aus der ersten, zweiten, dritten und vierten Intensität ($I_{Meß,MD}, I_{Ref,MD}, I_{Meß,RD}, I_{Ref,RD}$) ermittelt wird, und daß der Bräunungsgrad einer Ofensteuerung ( 60 ) zur Steuerung einer Wärmebehandlung zugeführt ist.

**16.** Verfahren nach Anspruch 15. bei dem das Meßsignal der vierten Intensität der Referenzstrahlung am Referenzsensor ( 24 ) ( $I_{Ref,RD}$) über eine solche Meßzeit integriert wird, bis ein fest vorgebbarer Wert des Integrals erreicht ist und das Meßsignal der ersten Intensität der Meßstrahlung am Meßdetektor (22) ($I_{Meß,MD}$) über die gleiche Meßzeit integriert wird und das zum Ende dieser Meßzeit erreichte Integral des Meßwerts der ersten. Intensität der Meßstrahlung ( $I_{Meß,MD}$) am Meßdetektor ( 22 ) zur Ermittlung des Bräunungsgrades herangezogen wird.

## Claims

**1.** Device for measuring the degree of browning of a cooking stock (14), particularly for measuring the degree of browning of a baking stock, with a cooking chamber (12), with at least one radiation source (16, 18; 82), which produces a measuring radiation, which is a radiation of a first wavelength range, and a reference radiation, which is a radiation of a second wavelength range different therefrom, the two being radiated into the cooking chamber (12), with a measuring sensor (22) for detecting a first intensity ($I_{Meas,MD}$) of radiation, which is picked up from the cooking chamber (12), in the first wavelength range and a second intensity ($I_{Ref,MD}$) of radiation, which is picked up from the cooking chamber (12), in the second wavelength range, and with a reference sensor (24), which is coupled with the radiation source (16, 18), for detection of a third intensity ($I_{Meas,RD}$) of radiation, which is radiated into the cooking chamber (12), in the first wavelength range and for detection of a fourth intensity ($I_{Ref,RD}$) of radiation, which is radiated into the cooking chamber (12), in the second wavelength range, and with equipment (52) for ascertaining the degree of browning from the first, second, third and fourth intensities ($I_{Meas,MD}, I_{Ref,MD}, I_{Meas,RD}, I_{Ref,RD}$) measured by the sensors (22, 24), wherein optical waveguides (30, 32, 34, 36; 84, 86, 88) for transmission of radiation from the radiation source (16, 18; 82) into the cooking chamber (12) and for transmission of radiation from the cooking chamber (12) to the measuring sensor (22) are provided, and with an oven control (60), to which the degree of browning is fed [to an oven control (60)] for control of a thermal treatment of the cooking chamber.

**2.** Device according to claim 1, in which the measuring radiation and reference radiation emitted by the radiation source (16, 18) is fed to a beam splitter (20) for splitting into two part beams (26, 28), wherein the first part beam (26) is fed to the reference sensor (22) for detection of the third and fourth intensities ($I_{Meas,RD}, I_{Ref,RD}$) and wherein the second part beam (28) of the measuring radiation and the reference radiation is radiated into the cooking chamber (12).

3. Device according to claim 2, in which the measurement signal, which is obtained from the reference sensor (24), of the third and fourth intensity ($I_{Meas,ID}$, $I_{Ref,MD}$) is fed to equipment (54) for regulation of the radiation intensities of the emitted measuring radiation and the emitted reference radiation to a constant value.

4. Device according to one of the preceding claims, in which the second part beam (28) of the reference radiation and the measuring radiation is fed by way of at least one optical waveguide (30, 32) to an optical system (40), from which the reference radiation is radiated, fanned out in one plane approximately at the level of the cooking stock (14), into the cooking chamber (12) and by which the backscattered radiation reflected from the cooking chamber (12) is fed to the measuring sensor (22) by way of at least one optical waveguide (36, 38) arranged substantially outside the radiation region of the measuring radiation and reference radiation incident in the cooking chamber (12).

5. Device according to claim 4, in which a respective optical waveguide (30, 32) is provided in order to connect the beam splitter (20) with the optical system (40), wherein the two optical waveguides (30, 32) are arranged to radiate in fanned-out manner into the cooking chamber (12) approximately centrally by way of the optical system (40) and wherein a respective optical waveguide (34, 36) is provided laterally in the edge regions of the cooking chamber (12) and feeds the backscattered radiation, which is reflected from the cooking chamber (12) and focussed by way of the optical system (40), to the measuring sensor (22).

6. Device according to one of the preceding claims, in which a linear light source array (82), the radiation of which is coupled into the cooking chamber (12) by way of an optical waveguide bundle (84), is provided as radiation source.

7. Device according to claim 6, in which a part of the radiation emitted by the light source array (82) is fed by way of a part fibre bundle (86) to a reference sensor (24), in which the rest of the emitted radiation is radiated into the cooking chamber (12) in widely fanned-out manner by way of the optical system (40) and in which the radiation focussed by the optical system (40) and reflected and backscattered from the cooking chamber (12) is fed by way of a second part fibre bundle (88) to the measuring sensor (22).

8. Device according to claim 6 or 7, in which the optical waveguide transmitting the reference radiation and the measuring radiation and the optical waveguide transmitting the reflected and backscattered radiation are arranged in linear array to be substantially uniformly distributed.

9. Device according to one of claims 4 to 8, in which the optical system (40) is constructed as a linear concentrating reflector or as a cylinder lens.

10. Device according to one of the preceding claims, in which the measuring radiation lies in the yellow visible range and the reference radiation lies in the infrared range.

11. Device according to claim 10, in which the measuring radiation and the reference radiation are produced by LEDs (16, 18), which emit in the wavelength range of approximately 600 nm and approximately 1000 nm.

12. Device according to claim 1 to 11, in which the oven control (60) causes a switching-off of heating equipment on attainment of a predetermined degree of browning.

13. Device according to one of the preceding claims, with equipment (60) for terminating the measurement time of the measuring radiation in dependence on the instant of reception of a presettable received radiation energy of the reference radiation, and with equipment (52) for integration of the measurement signal of the intensity of the measuring radiation at the measuring detector over the measurement time for determination of the measurement result.

14. Device according to one of the preceding claims, in which the reference radiation and the measuring radiation are modulated by different frequencies and are cyclically produced to be offset relative to one another in time, and in which the measurement signals picked up by the measuring sensor and the reference sensor are fed by way of a switching device (58) to a common amplifier and filter unit (56) for processing.

15. Method of measuring the degree of browning of a cooking stock (14), particularly for measuring the degree of browning of a baking stock, in the case of a thermal treatment in a cooking chamber (12), in which a measuring radiation, which is a radiation of a first wavelength range, and a reference radiation, which is a radiation of a second wavelength range different therefrom, the two being radiated into the cooking chamber (12), are produced by at least one radiation source (16, 18; 82), a first intensity ($I_{Meas,MD}$) of radiation, which is picked up from the cooking chamber (12), in the first wavelength range and a second intensity ($I_{Ref,MD}$) of radiation, which is picked up from the cooking chamber (12), in the second wavelength range, are detected by a measuring sensor (22), in which a third intensity ($I_{Meas,RD}$) of radiation, which is radiated into the cooking chamber (12), in the first wavelength range and a fourth intensity ($I_{Ref,RD}$) of radiation, which is radiated into the cooking chamber (12), in the second wavelength range, are detected by means of a reference sensor (24) coupled with the radiation source (16, 18), wherein the radiation between the cooking chamber (12) and the sensors (22, 24) is transmitted by way of optical waveguides (30, 32, 34, 36; 84, 86, 88) and wherein the degree of browning is ascertained from the first, second, third and fourth intensities ($I_{Meas,MD}$, $I_{Ref,MD}$, $I_{Meas,RD}$, $I_{Ref,RD}$) and that the degree of browning is fed to an oven control (60) for control of a thermal treatment.

16. Method according to claim 15, in which the measurement signal of the fourth intensity of the reference radiation at the reference sensor (24) ($I_{Ref,RD}$) is integrated over such a measurement time until a fixedly presettable value of the integral is reached and the measurement signal of the first intensity of the measuring radiation at the measuring detector (22)($I_{Meas,MD}$) is integrated over the same measurement time and the integral, which is reached at the end of this measurement time, of the measurement value of the first intensity of the measuring radiation ($I_{Meas,MD}$) at the measuring detector (22) is utilised for ascertaining the degree of browning.

**Revendications**

1. Dispositif pour la mesure du degré de cuisson d'un aliment à cuire (14), en particulier pour la mesure du degré de cuisson d'un produit de boulangerie avec un espace de cuisson (12), avec au moins une source de rayonnement (16, 18 ; 82), qui produit un rayonnement de mesure, qui est un rayonnement d'une première gamme d'ondes, et un rayonnement de référence qui est un rayonnement d'une deuxième gamme d'ondes, différente de celle-ci, qui sont irradiés l'un et l'autre dans l'espace de cuisson (12), avec un capteur de mesure (22) pour la saisie d'une première intensité ($I_{Meß,MD}$) du rayonnement dans la première gamme d'ondes enregistré à partir de l'espace de cuisson (12), et d'une deuxième intensité ($I_{Ref,MD}$) du rayonnement dans la deuxième gamme d'ondes enregistré à partir de l'espace de cuisson (12), et avec un capteur de référence (24) qui est couplé avec la source de rayonnement (16, 18) pour la saisie d'une troisième intensité ($I_{Meß,RD}$) du rayonnement dans la première gamme d'ondes irradié dans l'espace de cuisson (12) et pour la saisie d'une quatrième intensité ($I_{Ref,RD}$) du rayonnement dans la deuxième gamme d'ondes, irradié dans l'espace de cuisson (12), ainsi qu'avec un dispositif (52) pour la détermination du degré de cuisson d'après les première, deuxième, troisième et quatrième intensités ($I_{Meß,MD}$, $I_{Ref,MD}$, $I_{Meß,RD}$, $I_{Ref,RD}$) mesurées par les capteurs (22, 24), dans lequel des fibres optiques (30, 32, 34, 36 ; 84, 86, 88) sont prévues pour la transmission du rayonnement de la source de rayonnement (16, 18 ; 82) à l'espace de cuisson (12) et pour la transmission du rayonnement à partir de l'espace de cuisson (12) vers le capteur de mesure (22), et avec une commande de four (60) à laquelle le degré de cuisson est transmis pour la commande d'un traitement thermique de l'espace de cuisson.

2. Dispositif selon la revendication 1, dans lequel le rayonnement de mesure et le rayonnement de référence, émis par la source de rayonnement (16, 18), sont transmis à un séparateur de faisceaux (20) pour la division en deux faisceaux partiels (26, 28), dans lequel le premier faisceau partiel (26) est transmis au capteur de référence (22) pour la saisie de la troisième et de la quatrième intensités ($I_{Meß,RD}$, $I_{Ref,RD}$), et dans lequel le deuxième faisceau partiel (28) du rayonnement de mesure et du rayonnement de référence est irradié dans l'espace de cuisson (12).

3. Dispositif selon la revendication 2, dans lequel le signal de mesure de la troisième et de la quatrième intensités ($I_{Meß,RD}$, $I_{Ref,RD}$), reçu par le capteur de référence (24), est transmis à un dispositif (54) pour la régulation des intensités de rayonnement du rayonnement de mesure émis et du rayonnement de référence émis à une valeur constante.

4. Dispositif selon l'une quelconque des revendications précédentes, dans lequel le deuxième faisceau partiel (28) du rayonnement de référence et du rayonnement de mesure est transmis, par au moins une fibre optique (30, 32), à une optique (40) à partir de laquelle le rayonnement de référence est irradié à un niveau de façon dispersée approximativement à la hauteur de l'aliment à cuire (14), dans l'espace de cuisson (12), et à partir de laquelle le rayonnement réfléchi, rétrodiffusé à partir de l'espace de cuisson (12), est transmis au capteur de mesure (22) par au moins une fibre optique (36, 38) disposée pour l'essentiel en dehors de la zone de rayonnement du rayonnement de mesure et du rayonnement de référence incidents dans l'espace de cuisson (12)

**5.** Dispositif selon la revendication 4, dans lequel est prévu respectivement une fibre optique (30, 32) pour relier le séparateur de faisceaux (20) avec l'optique (40), dans lequel les deux fibres optiques (30, 32) sont disposés pour irradier dans l'espace de cuisson (12), de façon dispersée, approximativement au centre au-dessus de l'optique (40), et dans lequel, latéralement dans les zones marginales de l'espace de cuisson (12), est prévu à chaque fois une fibre optique (34, 36) qui transmet au capteur de mesure (22) le rayonnement concentré par l'optique (40), réfléchi, rétrodiffusé à partir de l'espace de cuisson (12).

**6.** Dispositif selon l'une quelconque des revendications précédentes, dans lequel est prévu comme source de rayonnement un réseau linéaire de sources lumineuses (82), dont le rayonnement est couplé dans l'espace de cuisson (12) par l'intermédiaire d'un faisceau de fibres optiques (84).

**7.** Dispositif selon la revendication 6, dans lequel une partie du rayonnement émis par le réseau de sources lumineuses (82) est transmis, par un faisceau partiel de fibres (86), à un capteur de référence (24), dans lequel le rayonnement émis résiduel est irradié, largement dispersé par l'optique (40), dans l'espace de cuisson (12), et dans lequel le rayonnement concentré par l'optique (40), réfléchi et rétrodiffusé à partir de l'espace de cuisson (12), est transmis au capteur de mesure (22) par un deuxième faisceau partiel de fibres (88).

**8.** Dispositif selon la revendication 6 ou 7, dans lequel les fibres optiques transmettant le rayonnement de référence et le rayonnement de mesure et les fibres optiques transmettant le rayonnement réfléchi et rétrodiffusé sont disposées, réparties avec régularité, en forme de lignes.

**9.** Dispositif selon l'une quelconque des revendications 4 à 8, dans lequel l'optique (40) est conçue en tant que réflecteur linéaire de concentration ou en tant que lentille cylindrique.

**10.** Dispositif selon l'une quelconque des revendications précédentes, dans lequel le rayonnement de mesure se situe dans le domaine visible jaune et le rayonnement de référence dans le domaine de l'infrarouge.

**11.** Dispositif selon la revendication 10, dans lequel le rayonnement de mesure et le rayonnement de référence sont produits par des DEL (16, 18) qui émettent dans la gamme de longueurs d'onde d'environ 600 nm et d'environ 1000 nm.

**12.** Dispositif selon les revendications 1-11, dans lequel la commande de four (60) provoque une mise hors tension d'un dispositif de chauffe lors de l'obtention d'un degré de cuisson pouvant être prédéterminé.

**13.** Dispositif selon l'une quelconque des revendications précédentes, avec un dispositif (60) pour la terminaison du temps de mesure du rayonnement de mesure en fonction du moment de réception d'une énergie rayonnante reçue pouvant être prédéterminée du rayonnement de référence, et avec un dispositif (52) pour l'intégration, au détecteur de mesure, du signal de mesure de l'intensité du rayonnement de mesure, sur le temps de mesure, pour la détermination du résultat de la mesure.

**14.** Dispositif selon l'une quelconque des revendications précédentes, dans lequel le rayonnement de référence et le rayonnement de mesure sont produits de manière cadencée, modulés avec des fréquences différentes et décalés dans le temps l'un par rapport à l'autre, et dans lequel les signaux de mesure enregistrés par le capteur de mesure et le capteur de référence sont transmis pour traitement, par un dispositif de commutation (58), à une unité commune d'amplificateur et de filtre (56).

**15.** Procédé pour la mesure du degré de cuisson d'un aliment à cuire (14), en particulier pour la mesure du degré de cuisson d'un produit de boulangerie, pendant un traitement thermique dans un espace de cuisson (12), dans lequel sont produits, par au moins une source de rayonnement (16, 18 ; 82), un rayonnement de mesure, qui est un rayonnement d'une première gamme d'ondes, et un rayonnement de référence qui est un rayonnement d'une deuxième gamme d'ondes, différente de celle-ci, qui sont irradiés l'un et l'autre dans l'espace de cuisson (12), dans lequel un capteur de mesure (22) saisit une première intensité ($I_{Meß,MD}$) du rayonnement dans la première gamme d'ondes enregistré à partir de l'espace de cuisson (12) et une deuxième intensité ($I_{Ref,MD}$) du rayonnement dans la deuxième gamme d'ondes enregistré à partir de l'espace de cuisson (12), dans lequel, au moyen d'un capteur de référence (24) couplé avec la source de rayonnement (16, 18 ; 82), sont saisies une troisième intensité ($I_{Meß,RD}$) du rayonnement dans la première gamme d'ondes irradié dans l'espace de cuisson (12) et une quatrième intensité ($I_{Ref,RD}$) du rayonnement dans la deuxième gamme d'ondes, irradié dans l'espace de cuisson (12), dans lequel le rayonnement entre l'espace de cuisson (12) et les capteurs (22, 24) sont transmis par des fibres optiques (30, 32, 34, 36 ; 84, 86, 88) et dans lequel le degré de cuisson est déterminé d'après les première, deuxième, troisième et quatrième intensités ($I_{Meß,MD}$, $I_{Ref,MD}$, $I_{Meß,RD}$, $I_{Ref,RD}$) et le degré de cuisson est transmis à une commande de four (60) pour la commande d'un traitement thermique.

16. Procédé selon la revendication 15, dans lequel le signal de mesure de la quatrième intensité du rayonnement de référence est intégré au capteur de référence (24) ($I_{Ref,RD}$) sur un temps de mesure jusqu'à ce qu'une valeur pouvant être prédéterminée fixement de l'intégrale soit atteinte et le signal de mesure de la première intensité du rayonnement de mesure est intégré au détecteur de mesure (22) ($I_{Meß,MD}$) sur la même durée de mesure et l'intégrale, atteinte à la fin de cette durée de mesure, de la valeur mesurée de la première intensité du rayonnement de mesure ($I_{Meß,MD}$) sur le détecteur de mesure (22) est prise en compte pour la détermination du degré de cuisson.

Fig. 1

Fig. 2

Fig. 3

Bräunungsgrad Bretzeln

Minuten

Fig. 4

EP 0 682 243 B1

Bräunungsgrad Spitzbrötchen

Minuten

Fig. 5

Bräunungsgrad Laugenbrötchen

Minuten

Fig. 6

EP 0 682 243 B1